# DEMANDE DE BREVET EUROPEEN

(11) **EP 2 111 874 A1**
(43) Date de publication de la demande: **28.10.2009**
(21) Numéro de dépôt: 09157529.0
(22) Date de dépôt: 07.04.2009
(51) Int. Cl.: A61L 2/22, A61L 2/24

(54) **Machine de désinfection de surfaces par voie aérienne**

(30) Priorité: 18.04.2008 FR 0852661
(71) Demandeur: Hygiatech, 92250 La Garenne Colombes (FR)
(72) Inventeur: Gavard, Jean, 92250, La Garenne Colombes (FR)
(74) Mandataire: Chaillot, Geneviève

(57) **Abrégé**

La présente invention a pour objet une machine
(1) de désinfection par voie aérienne des surfaces d'une enceinte fermée, comprenant :
- un bidon (16) de produit à atomiser ;
- un atomiseur (18) ;
- des moyens (14, 21) permettant d'alimenter l'atomiseur (18) en produit à atomiser ;
- des moyens (24) d'alimentation électrique des différents composants de la machine (1) ;

caractérisée par le fait qu'elle comprend en outre des moyens de calcul et de mémoire (17) avec une interface permettant de programmer un fonctionnement automatique de la machine (1). Les moyens de calcul et de mémoire peuvent comprendre un programme permettant d'avoir une traçabilité de l'utilisation de la machine (1).

## Description

La présente invention a pour objet une machine de désinfection de surfaces par voie aérienne dans une enceinte, par pulvérisation dans l'enceinte à désinfecter d'une solution chargée de matières actives possédant des propriétés désinfectantes.

Il est connu de l'état antérieur de la technique, et en particulier du brevet européen EP0291616, des machines pour désinfecter des surfaces dans des enceintes, par pulvérisation dans l'enceinte d'une solution désinfectante.

Ces machines présentent plusieurs inconvénients. Tout d'abord, il n'est pas possible de programmer automatiquement des opérations de désinfection. Ensuite, il est important, pour les désinfections de salles, par exemple de salles blanches, de pouvoir conserver une traçabilité des opérations de désinfection réalisées, des opérateurs ayant pratiqué la désinfection, et une garantie d'une quantité de poids de produit diffusé.

Aucune machine de l'état antérieur de la technique ne présente ces caractéristiques d'automatisation de l'opération de désinfection et de traçabilité des opérations de désinfection.

La présente invention permet de pallier ces inconvénients, et a pour objet une machine dotée de moyens permettant de programmer des opérations de désinfection, de maintenir une traçabilité des opérations de désinfection réalisées et de contrôler en temps réel le poids du produit diffusé.

En particulier, la machine selon l'invention permet une traçabilité conforme à la réglementation américaine (FDA) 21 CFR part 11 sur les signatures électroniques.

La présente invention a donc pour objet une machine de désinfection par voie aérienne des surfaces d'une enceinte fermée, comprenant :
- un bidon ou réservoir de produit à atomiser ;
- un atomiseur ;
- des moyens permettant d'alimenter l'atomiseur en produit à atomiser ;
- des moyens d'alimentation électrique des différents composants de la machine ;
caractérisée par le fait qu'elle comprend en outre des moyens de calcul et de mémoire avec une interface permettant de programmer un fonctionnement automatique de la machine.

Les moyens de calcul et de mémoire peuvent être par exemple un automate, avec une interface d'écran et un pavé de touche pour la programmation de l'automate.

La machine peut comprendre en outre des moyens permettant la lecture d'un identifiant codé d'un opérateur de la machine, d'une enceinte à traiter, et d'un produit utilisé pour la désinfection et le stockage dans les moyens de calcul et de mémoire des identifiants codés lus.

L'identifiant codé peut être un code à barres.

La machine peut comprendre en outre des moyens de mesure de la quantité de produit restante dans le bidon de produit à atomiser, lesdits moyens de mesure étant interfacés avec lesdits moyens de calcul et de mémoire.

La machine peut également comprendre en outre des moyens de mesure de l'hygrométrie dans l'enceinte à traiter.

La machine peut également comprendre en outre des indicateurs visuels commandés par un programme mis en oeuvre dans les moyens de calcul et de mémoire pour indiquer à un opérateur à l'extérieur de l'enceinte la phase de traitement en cours de la machine.

Le programme peut être apte à empêcher un fonctionnement de la machine lorsque l'identifiant codé lu de l'opérateur, de l'enceinte ou du produit n'est pas conforme à un identifiant stocké dans les moyens de calcul et de mémoire.

Les quantités de produit nécessaires pour chaque enceinte à traiter peuvent être stockées dans les moyens de calcul et de mémoire et que le programme peut être apte à empêcher le fonctionnement de la machine lorsque la quantité de produit restante mesurée par les moyens de mesure de la quantité de produit restante dans le bidon est inférieure à la quantité nécessaire stockée dans les moyens de calcul et de mémoire pour l'enceinte à traiter.

Le programme peut également être apte à empêcher le fonctionnement de la machine lorsque le taux d'hygrométrie mesuré par les moyens de mesure du taux d'hygrométrie ne correspond pas à un taux d'hygrométrie de référence stocké dans les moyens de calcul et de mémoire.

Les moyens de calcul et de mémoire peuvent comprendre des moyens d'horloge pour différer un fonctionnement de la machine.

Pour chaque traitement, le programme peut être apte à enregistrer dans les moyens de calcul et de mémoire l'opérateur de la machine, l'enceinte traitée, la date, la durée de traitement, et la quantité de produit utilisée pour le traitement.

Le programme peut également être apte à modifier une quantité de produit atomisé par l'atomiseur conformément à un type de traitement choisi par l'opérateur au moyen de l'interface des moyens de calcul et de mémoire.

L'atomiseur peut être un atomiseur en résine thermoplastique au méthacrylate de méthyle (lucite^{®}).

La présente invention a également pour objet un procédé de désinfection de surfaces dans une enceinte fermée par voie aérienne, mis en oeuvre par la machine telle que définie ci-dessus, caractérisé par le fait qu'il comprend les étapes suivantes :
- l'opérateur s'identifie sur la machine par lecture, avec les moyens permettant la lecture d'un identifiant codé, d'un code à barres qui lui est associé ;
- l'opérateur identifie l'enceinte à traiter par lecture, avec les moyens permettant la lecture d'un identifiant codé, d'un code à barres associé à l'enceinte ;
- l'opérateur identifie le produit de désinfection utilisé pour le traitement par lecture, avec les moyens permettant la lecture d'un identifiant codé, d'un code à barres associé au produit de désinfection utilisé ;
- le programme mis en oeuvre dans les moyens de calcul et de mémoire vérifie les identifiants lus de l'opérateur, de l'enceinte, et du produit utilisé et, par comparaison avec des données en mémoire dans les moyens de calcul et de mémoire concernant l'opérateur, l'enceinte et le produit utilisé, autorise ou non le fonctionnement de la machine ;
- l'opérateur met en place le bidon de produit de désinfection utilisé dans la machine ;
- le programme mis en oeuvre dans les moyens de calcul et de mémoire vérifie, par l'intermédiaire des moyens de mesure, si la quantité de produit de désinfection utilisé est suffisante pour le traitement de l'enceinte, par comparaison de la quantité restante de produit mesuré avec des données en mémoire dans les moyens de calcul et de mémoire concernant l'enceinte, et empêche le fonctionnement de la machine si la quantité restante de produit est insuffisante ;
- l'opérateur programme, avec l'interface des moyens de calcul et de mémoire, le fonctionnement de la machine ; et
- le programme enregistre en mémoire, à la fin du traitement, l'opérateur, l'enceinte, la date, la durée et la quantité de produit utilisée pour le traitement.

Pour mieux illustrer la machine de désinfection de surfaces par voie aérienne selon la présente invention, on va en décrire ci-après un mode de réalisation particulier, avec référence au dessin annexé.

Sur ce dessin :
- la Figure 1 est une vue d'ensemble en perspective de la machine selon l'invention ;
- la Figure 2 est une vue de côté de la machine de la Figure 1, montrant l'intérieur de la machine ;
- la Figure 3 est une vue éclatée de la machine de la Figure 1, montrant ses différents éléments constitutifs.

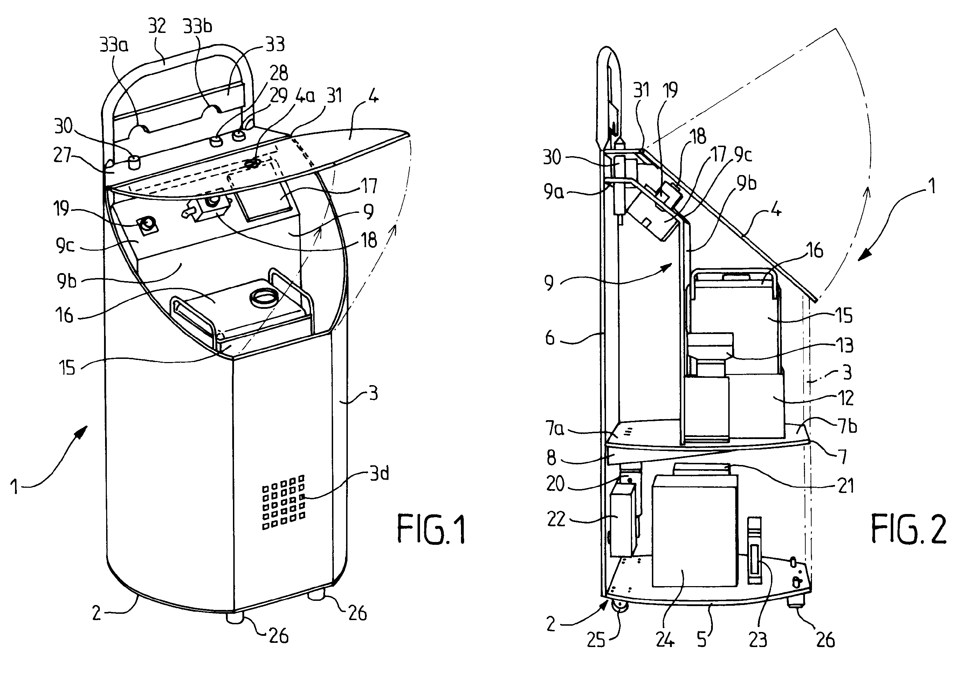

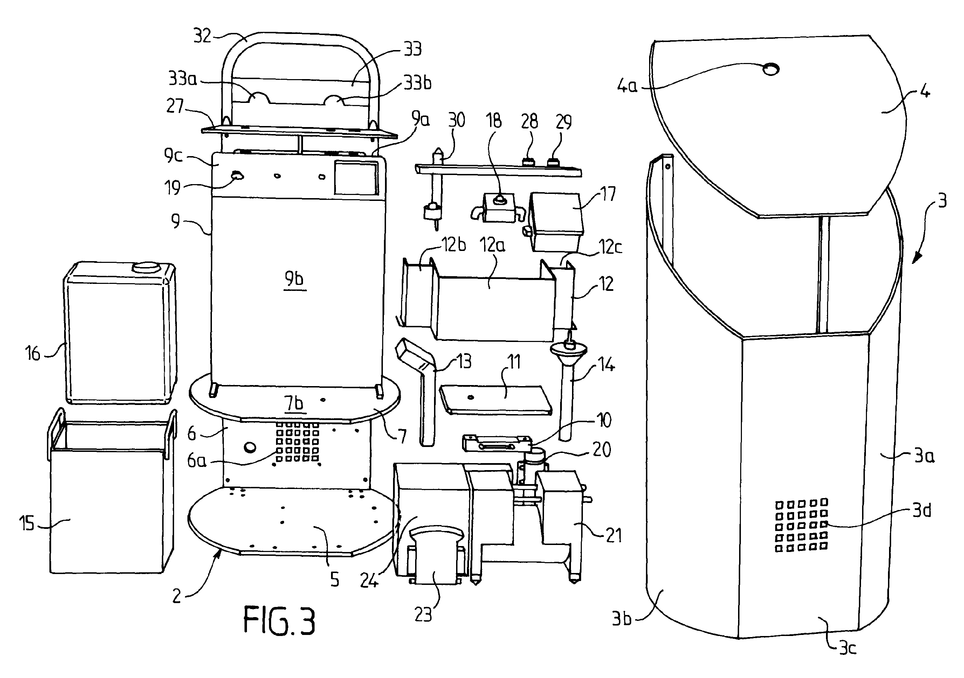

Si l'on se réfère aux Figures 1 à 3, on peut voir que la machine 1 est constituée d'un châssis 2, d'un capot 3 et d'une trappe 4. Le châssis 2 comporte une base 5 à bords latéraux sensiblement arrondis, et une paroi verticale 6 de forme sensiblement rectangulaire située sur le bord arrière de la base 5, lorsque l'on regarde la Figure 3. Le châssis est formé à partir d'une feuille d'acier inoxydable (304L), pliée suivant la forme d'un L.

La base 5 définit une première étagère inférieure, et une étagère supérieure 7, parallèle et de même forme que la base 5, est portée par la face avant, toujours lorsque l'on regarde la Figure 3, de la paroi verticale 6 du châssis 2 par l'intermédiaire d'équerres 8 fixées sur la paroi verticale 6 du châssis 2.

Une pièce de séparation 9, également en acier inoxydable, de même largeur que la paroi verticale 6, et comportant une face horizontale 9a, une face verticale 9b, et une face inclinée 9c reliant la face horizontale 9a et la face verticale 9b est soudée à la paroi verticale 6 du châssis 2 par le bord libre de la face horizontale 9a, et est soudée à la face supérieure de l'étagère supérieure 7 par le bord libre de la face verticale 9b.

La pièce de séparation 9 sépare l'étagère supérieure 7 en une partie arrière 7a située entre la paroi verticale 6 du châssis 2 et la pièce de séparation 9, et une partie avant 7b correspondant à la partie de l'étagère supérieure 7 située devant la pièce de séparation 9, lorsque l'on regarde la Figure 3.

Un capteur de poids 10 est fixé sur sa partie basse par deux vis sur la partie avant 7b de l'étagère supérieure 7. Une plaque 11 en acier inoxydable est fixée par deux autres vis sur la partie haute du capteur de poids 10.

Une pièce de maintien 12, également en acier inoxydable, comportant trois compartiments rectangulaires 12a, 12b, 12c, est soudée par ses bords libres à la face avant, lorsque l'on regarde la Figure 3, de la face verticale 9b de la pièce de séparation 9, sur la partie avant 7b de l'étagère supérieure 7.

Le premier compartiment 12a, central, correspond à la surface de la plaque 11, les deux autres compartiments latéraux 12b, 12c, plus petits que le compartiment 12a, et situés sur les côtés du compartiment 12a, permettent respectivement de ranger une douchette de lecture de code à barres 13, et une canne d'aspiration ou sonde de niveau 14.

En utilisation, un bac porte-bidon 15 recevant un bidon 16 contenant le produit à pulvériser, est disposé dans le compartiment central 12a de la pièce de maintien 12.

La face inclinée 9c porte un automate 17, un atomiseur en résine thermoplastique au méthacrylate de méthyle 18, qui permet l'atomisation du produit contenu dans le bidon 16 en brumisation, et un connecteur 19 pour la douchette à code à barres 13 et la communication avec l'extérieur de l'automate 17.

La canne d'aspiration 14 est disposée, en utilisation, dans le bidon 16 et permet d'aspirer le produit à pulvériser, qui est adressé, par l'intermédiaire d'un canal (non représenté) à l'atomiseur 18, pour une distribution du produit dans le bidon 16 par l'atomiseur 18.

L'atomiseur en résine thermoplastique au méthacrylate de méthyle 18 permet l'atomisation du produit en brumisation. Sa matière permet par ses propriétés translucides d'effectuer un contrôle visuel de l'état des surfaces après brumisation. Son fonctionnement consiste à diffuser en fines gouttelettes (taille entre 20 à 25µ) le produit biocide. Cette diffusion s'obtient par l'aspiration du produit créée par le passage de la pression de l'air dans l'atomiseur 18. La veine de produit se fractionne sous la pression de l'air dans la buse de diffusion et forme de fines gouttelettes créant ainsi un brouillard sec. La production de l'air est gérée par l'automate 17.

Sur la base 5 du châssis 2 sont fixés un filtre régulateur de pression 20, un compresseur 21, un ventilateur 22, un conditionneur de signal analogique 23 et des éléments électriques 24 permettant l'alimentation des différents composants de la machine 1. Cette partie électrique est sécurisée par un disjoncteur permettant en cas de surtension de couper l'alimentation de la machine 1.

Le compresseur 21 permet une production d'air avec une pression suivant le volume d'air produit. Ce compresseur 21 sans bain d'huile est équipé d'un moteur électrique asynchrone permettant de ne pas rejeter de particules dans l'atmosphère lors de son fonctionnement.

L'air produit est dirigé vers le régulateur de pression 20 à décharge automatique, équipé d'une filtration à 25µ. L'air ainsi régulé est adressé par un flexible (non représenté) à l'atomiseur 18.

Le conditionneur de signal analogique 23 est relié à l'entrée analogique de l'automate 17 afin de pouvoir gérer et enregistrer cette information dans le programme interne de l'automate 17. Une alimentation électrique 24 pour les différents composants de la machine 1 permet également de mettre en sûreté la machine 1 en cas de surtension électrique.

Des perforations 6a dans la paroi verticale 6 du châssis 2, entre l'étagère supérieure 7 et la base 5 du châssis 2, permettent un meilleur fonctionnement du ventilateur 22.

Deux roulettes 25 et deux supports de machine ou pieds 26 sont respectivement montés à l'arrière et à l'avant de la machine 1, sur la face inférieure de la base 5.

Une plaque supérieure 27, parallèle à la face horizontale 9a de la pièce de séparation 9, et de largeur supérieure, est soudée sur la face avant, lorsque l'on regarde la Figure 3, de la paroi verticale 6 du châssis 2 au-dessus de la face horizontale 9a.

La plaque supérieure 27 porte sur sa face supérieure deux voyants lumineux 28, 29, un rouge 28 et un vert 29, ainsi qu'une sonde hygrométrique 30.

Le bord de la plaque supérieure 27 opposé à celui fixé à la paroi verticale 6 du châssis 2 porte une charnière 31 reliée à la trappe 4, la charnière 31 permettant d'ouvrir et de fermer la trappe 4, reposant, lorsqu'elle est fermée, sur le capot 3 de la machine 1 à un angle de 40°.

Le conditionneur de signal analogique 23 du capteur de poids 10, le connecteur de douchette 19, les voyants lumineux 28, 29, la sonde hygrométrique 30, le compresseur 21 sont reliés à l'automate 17 qui les commande et à l'alimentation électrique 24 par des fils de connexion non représentés.

Le capot machine 3 est constitué de deux profilés 3a, 3b à coupe transversale arrondie, biseautés à une de leurs extrémités.

Les deux profilés 3a, 3b sont réunis par une plaque métallique 3c rectangulaire au niveau de leur bord le plus court.

Les bords les plus longs du capot 3 de la machine 1 sont fixés à la face arrière, sur la Figure 3, de la paroi latérale 6 du châssis 2, tandis que la partie inférieure du capot 3 de la machine 1 est fixée sur la face supérieure de la base 5 du châssis 2.

Lorsque le capot 3 de la machine 1 est monté sur le châssis 2, la forme en coupe transversale de l'ensemble paroi verticale 6 de châssis 2-capot 3 de la machine 1 correspond sensiblement à la forme de l'étagère supérieure 7 et de la base 5.

La plaque métallique rectangulaire 3c verticale du capot 3 de la machine 1 comporte des perforations 3d, au niveau de sa partie située entre la base 5 et l'étagère supérieure 7, pour le bon fonctionnement du ventilateur 22 et un refroidissement du compresseur 21.

La forme du capot 3 de la machine 1, ainsi que l'inclinaison de la trappe 2, sont choisis afin d'avoir le dépôt particulaire le plus faible possible sur la machine 1 et une meilleure diffusion dans l'espace à traiter.

La trappe 4 a sensiblement la forme de l'ouverture du capot 3 de la machine 1, lorsque le capot 3 est monté sur le châssis 2.

La trappe 4 comporte en outre un trou 4a, se positionnant au-dessus de l'atomiseur 18 lorsque la trappe 4 est fermée sur le capot 3, pour permettre une pulvérisation du produit de désinfection.

Une barre 32, sensiblement en forme de U retourné, sert de poignée, et est montée sur la partie supérieure de la paroi verticale 6 du châssis 2. Elle permet de faire basculer la machine 1 pour la déplacer sur ses roulettes 25.

Une barre horizontale 33, fixée entre les deux montants verticaux de ladite poignée 32 et portant sur son bord inférieur deux évidements 33a et 33b semi-circulaires espacés, qui permettent de fixer la machine 1 à un mur d'une enceinte.

L'automate 17 et son programme informatique commandent les fonctions de la machine 1, et notamment la diffusion du produit par l'atomiseur par l'intermédiaire du déclenchement du compresseur 21.

L'automate comprend des moyens de calcul, des moyens de mémoire et des moyens d'interface avec les différents éléments de la machine 1, et peut s'interfacer avec un ordinateur.

Dans la mémoire sont enregistrées au moins cinq bases de données différentes :
- une base de données pour les opérateurs de la machine ;
- une base de données des pièces que la machine peut traiter ;
- une base de données pour les valeurs de diffusions du produits ;
- une base de données pour les différents paramètres techniques de la machine ;
- une base de données regroupant les différentes données de la traçabilité de chaque traitement effectué par la machine.

Un programme enregistré dans l'automate 17 guide l'opérateur lors des différentes étapes de l'utilisation de la machine 1, par un affichage graphique sur un écran disposé sur l'automate 17.

Un pavé de touches lié à l'automate permet à l'utilisateur d'interagir avec l'automate 17 et de commander ses différentes fonctions.

L'automate 17 enregistre lors de son fonctionnement, l'opérateur, la pièce, l'identité du bidon, le poids du produit en temps réel, l'hygrométrie en début et fin de diffusion détectée par la sonde, l'heure de démarrage et d'arrêt de traitement. L'automate 17 commande également les voyants lumineux 28, 29, le compresseur 21 et l'affichage des divers éléments de mesures.

Un affichage et un contrôle du taux d'hygrométrie sont réalisés dans la phase de démarrage de la machine 1. Un seuil de 70% du taux d'hygrométrie bloque le fonctionnement de la machine 1, garantissant de cette façon une efficacité du traitement.

La douchette de lecture de code à barres 13 est connectée à l'automate 17 par un câble (non représenté) et une prise reliés au connecteur 19. Ce connecteur 19 est relié au port interface de l'automate 17.

La machine 1 est tout d'abord branchée par l'opérateur, dans une pièce à traiter.

Dans une première phase, l'opérateur s'identifie par la lecture de son code à barres par utilisation de la douchette 13. Le code à barres lu est comparé à un code à barres stocké dans une base de données en mémoire de l'automate 17, dans laquelle sont également stockés les données de l'opérateur.

Un code de validation et une position hiérarchique de l'opérateur peuvent être inscrits.

Une caractéristique de hiérarchie entre les opérateurs peut en effet être prévue dans l'automate 17, permettant à un opérateur plus haut dans la hiérarchie de modifier le traitement programmé par un opérateur plus bas dans la hiérarchie.

Si le code à barres ne correspond à aucun code enregistré, l'opérateur n'est pas reconnu et ne peut pas utiliser la machine.

Si le code à barres correspond à un code à barres enregistré dans la base de données de l'automate 17, l'opérateur est reconnu et son nom est affiché sur l'écran de l'automate 17.

L'opérateur doit alors valider son authentification par l'entrée, à l'aide du pavé de touches de l'automate 17, d'un code de validation d'identification d'opérateur. Ce code est comparé au code associé en mémoire à l'identifiant d'opérateur.

Si le code entré par l'opérateur correspond au code enregistré en mémoire, l'opérateur peut poursuivre les opérations, sinon, il ne peut pas poursuivre le traitement avec la machine 1.

Dans une seconde phase, l'opérateur choisit la pièce à traiter : les pièces sont enregistrées en mémoire, avec pour chaque pièce un volume de traitement et un code à barres associé.

L'opérateur lit avec la douchette 13 le code à barres de la pièce à traiter, et l'automate 17 affiche alors le nom de la pièce et son volume.

Une fois la pièce choisie, l'opérateur choisit le traitement qu'il souhaite appliquer à la pièce, suivant différents types de traitement disponibles enregistrés dans une base de données en mémoire, par exemple, trois traitement différents : préventif, curatif ou intensif, correspondant à différentes quantités en g/m³ de produit à atomiser dans la pièce.

Ce choix est fait par l'intermédiaire du pavé de touches de l'automate 17. Une fois le choix effectué par l'opérateur, l'automate 17 affiche le traitement choisi. L'opérateur doit ensuite valider le choix de traitement par le pavé de touches de l'automate 17.

L'automate calcule alors, en fonction du volume de la pièce identifiée et de la quantité en gr/m3 choisie, la quantité de produit nécessaire, et affiche à la fois la quantité nécessaire de produit et la quantité restante.

Si la quantité restante est inférieure à la quantité nécessaire, on ne peut pas valider le démarrage de la machine 1. Il faut changer le bidon 16 de produit en appuyant sur une touche définie pour permettre à l'opérateur de lire avec la douchette 13 le nouveau code à barres du nouveau bidon 16.

Le code racine du nouveau code à barres du bidon est alors comparé avec un paramètre en mémoire interne. Après validation du code racine, le nouveau code à barres est enregistré dans la mémoire de l'automate 17.

Si le bidon 16 n'est pas reconnu, l'automate 17 revient à l'étape de lecture du code à barres de bidon.

Si le code est correct, l'automate 17 affiche la quantité du bidon 16 et la quantité nécessaire pour le traitement de la pièce.

Si la quantité restante est supérieure à la quantité nécessaire, on peut valider le démarrage de la machine 1, qui peut être différé par programmation grâce à une horloge interne à l'automate 17 à une certaine heure et à une certaine date. La validation immédiate entraîne un démarrage immédiat.

Dans le cas d'un démarrage immédiat, l'opérateur a alors une quantité de temps pour sortir de la pièce, et l'automate 17 affiche le temps restant avant la fin du traitement.

Les voyants 28, 29 permettent aux opérateurs de connaître l'état d'avancement du traitement depuis l'extérieur de la pièce, sans avoir à rentrer dans la pièce en traitement.

Un voyant vert 29 clignotant indique que le démarrage de la machine est programmé mais que le traitement n'a pas encore démarré.

Un voyant rouge 28 continu indique que le traitement est en cours.

Un voyant rouge 28 clignotant indique que le produit a été diffusé par l'atomiseur, et qu'il est en contact avec les surfaces à traiter. Personne ne peut alors rentrer dans la salle à traiter.

Une fois que le traitement est terminé, le voyant vert 29 s'allume de manière continue, indiquant à l'opérateur qu'il peut remettre en fonction le traitement de l'air de la pièce ou rentrer dans la salle avec protection pour remettre en marche de façon manuelle la ventilation de la pièce.

Tous les paramètres du traitement (opérateur, bidon produit utilisé, quantité de produit utilisé, date, heure de début de traitement, heure de fin de traitement, pièce, durée de traitement, hygrométrie début et fin) sont enregistrés en mémoire, permettant d'avoir en mémoire une traçabilité de chaque traitement, conformément à la réglementation américaine 21 CFR part 11.

Toutes les actions prises lors du traitement sont alors enregistrées.

On peut également transférer les données de traitement enregistrées sur la machine vers une base de données externe, par l'intermédiaire d'une liaison vers un ordinateur, permettant ainsi de conserver un journal des traitements des pièces.

Des moyens permettent de constater une anomalie pendant le traitement, par exemple, si une coupure électrique se produit pendant le traitement ou s'il y a un problème avec le produit, aucune heure de fin n'est enregistrée en mémoire, indiquant à l'opérateur que le traitement ne s'est pas déroulé correctement, et lui permettant de prendre des mesures correctives.

## Revendications

1. Machine (1) de désinfection par voie aérienne des surfaces d'une enceinte fermée, comprenant :
- un bidon (16) de produit à atomiser ;
- un atomiseur (18) ;
- des moyens (14, 21) permettant d'alimenter l'atomiseur (18) en produit à atomiser ;
- des moyens (24) d'alimentation électrique des différents composants de la machine (1) ;
- des moyens de calcul et de mémoire (17) avec une interface permettant de programmer un fonctionnement automatique de la machine (1),
**caractérisée par le fait qu'**elle comprend en outre des moyens (13, 19) permettant la lecture d'un identifiant codé d'un opérateur de la machine (1), d'une enceinte à traiter, et d'un produit utilisé pour la désinfection et le stockage dans les moyens de calcul et de mémoire (17) des identifiants codés lus.

2. Machine (1) selon la revendication 1,
**caractérisée par le fait que** l'identifiant codé est un code à barres.

3. Machine (1) selon l'une des revendications 1 à 2, **caractérisée par le fait qu'**elle comprend en outre des moyens (10, 23) de mesure de la quantité de produit restante dans le bidon (16) de produit à atomiser, lesdits moyens (10, 23) de mesure étant interfacés avec lesdits moyens de calcul et de mémoire (17).

4. Machine (1) selon l'une des revendications 1 à 3, **caractérisée par le fait qu'**elle comprend en outre des moyens (30) de mesure de l'hygrométrie dans l'enceinte à traiter.

5. Machine (1) selon l'une des revendications 1 à 4, **caractérisée par le fait qu'**elle comprend en outre des indicateurs visuels (28, 29) commandés par un programme mis en oeuvre dans les moyens de calcul et de mémoire (17) pour indiquer à un opérateur à l'extérieur de l'enceinte la phase de traitement en cours de la machine (1).

6. Machine (1) selon l'une des revendications 1 à 5, **caractérisée par le fait que** le programme est apte à empêcher un fonctionnement de la machine (1) lorsque l'identifiant codé lu de l'opérateur, de l'enceinte ou du produit n'est pas conforme à un identifiant stocké dans les moyens de calcul et de mémoire (17).

7. Machine (1) selon l'une des revendications 5 à 6, **caractérisée par le fait que** les quantités de produit nécessaires pour chaque enceinte à traiter sont stockées dans les moyens de calcul et de mémoire (17) et que le programme est apte à empêcher le fonctionnement de la machine (1) lorsque la quantité de produit restante mesurée par les moyens (10, 23) de mesure de la quantité de produit restante dans le bidon (16) est inférieure à la quantité nécessaire stockée dans les moyens de calcul et de mémoire (17) pour l'enceinte à traiter.

8. Machine (1) selon la revendication 4 ou l'une des revendications 5 à 7, lorsqu'elles dépendent de la revendication 4, **caractérisée par le fait que** le programme est apte à empêcher le fonctionnement de la machine lorsque le taux d'hygrométrie mesuré par les moyens (30) de mesure du taux d'hygrométrie ne correspond pas à un taux d'hygrométrie de référence stocké dans les moyens de calcul et de mémoire (17).

9. Machine (1) selon l'une des revendications 1 à 8, **caractérisée par le fait que** les moyens de calcul et de mémoire (17) comprennent des moyens d'horloge pour différer un fonctionnement de la machine.

10. Machine (1) selon la revendication 9,
**caractérisée par le fait que** pour chaque traitement, le programme est apte à enregistrer dans les moyens de calcul et de mémoire (17) l'opérateur de la machine (1), l'enceinte traitée, la date, la durée de traitement, et la quantité de produit utilisée pour le traitement.

11. Machine (1) selon l'une des revendications 5 à 10, **caractérisée par le fait que** le programme est apte à modifier une quantité de produit atomisé par l'atomiseur conformément à un type de traitement choisi par l'opérateur au moyen de l'interface des moyens de calcul et de mémoire (17).

12. Machine (1) selon l'une des revendications 1 à 10, **caractérisée par le fait que** l'atomiseur (18) est un atomiseur en résine thermoplastique au méthacrylate de méthyle.

13. Procédé de désinfection de surfaces dans une enceinte fermée par voie aérienne, mis en oeuvre par la machine (1) telle que revendiquée à la revendication 11 ou 12, **caractérisé par le fait qu'**il comprend les étapes suivantes :
- l'opérateur s'identifie sur la machine (1) par lecture, avec les moyens (13, 19) permettant la lecture d'un identifiant codé, d'un code à barres qui lui est associé ;
- l'opérateur identifie l'enceinte à traiter par lecture, avec les moyens (13, 19) permettant la lecture d'un identifiant codé, d'un code à barres associé à l'enceinte ;
- l'opérateur identifie le produit de désinfection utilisé pour le traitement par lecture, avec les moyens (13, 19) permettant la lecture d'un identifiant codé, d'un code à barres associé au produit de désinfection utilisé ;
- le programme mis en oeuvre dans les moyens de calcul et de mémoire (17) vérifie les identifiants lus de l'opérateur, de l'enceinte, et du produit utilisé et, par comparaison avec des données en mémoire dans les moyens de calcul et de mémoire (17) concernant l'opérateur, l'enceinte et le produit utilisé, autorise ou non le fonctionnement de la machine ;
- l'opérateur met en place le bidon (16) de produit de désinfection utilisé dans la machine (1) ;
- le programme mis en oeuvre dans les moyens de calcul et de mémoire (17) vérifie, par l'intermédiaire des moyens de mesure (10, 23), si la quantité de produit de désinfection utilisé est suffisante pour le traitement de l'enceinte, par comparaison de la quantité restante de produit mesuré avec des données en mémoire dans les moyens de calcul et de mémoire (17) concernant l'enceinte, et empêche le fonctionnement de la machine si la quantité restante de produit est insuffisante ;
- l'opérateur programme, avec l'interface des moyens de calcul et de mémoire (17), le fonctionnement de la machine ; et
- le programme enregistre en mémoire, à la fin du traitement, l'opérateur, l'enceinte, la date, la durée et la quantité de produit utilisée pour le traitement.
